# FASCICULE DE BREVET EUROPEEN

(11) **EP 3 071 212 B1**
(45) Date de publication et mention de la délivrance du brevet: **15.04.2020**
(21) Numéro de dépôt: 14799180.6
(22) Date de dépôt: 18.11.2014
(51) Int. Cl.: A61K 36/05, A61P 37/02

(54) **EXTRAIT D'ALGUES POUR SON UTILISATION EN TANT QU'AGENT IMMUNOMODULATEUR**
EXTRAKT AUS ALGEN ZUR VERWENDUNG ALS IMMUNMODULATOR
EXTRACT OF ALGAE FOR USE AS AN IMMUNOMODULATORY AGENT

(30) Priorité: 18.11.2013 FR 1361293
(43) Date de publication de la demande: 28.09.2016
(73) Titulaire: Amadeite, 56580 Brehan (FR)
(72) Inventeur: DEMAIS, Hervé, F-56700 Merlevenez (FR); NYVALL COLLÈN, Pi, F-29680 Roscoff (FR); LE GOFF, Matthieu, F-56800 Ploermel (FR); LE CHEVILLER, Isabelle, F-56580 Bréhan (FR)
(74) Mandataire: Lavoix
(86) Numéro de dépôt international: PCT/EP2014/074937
(87) Numéro de publication internationale: WO 2015/071497

(56) Documents cités:
- WO-A1-2010/115149
- WO-A1-2014/076261
- CN-A- 102 742 907
- US-A1- 2004 208 893
- CASTRO R ET AL: "Stimulation of turbot phagocytes by Ulva rigida C. Agardh polysaccharides", AQUACULTURE, ELSEVIER, AMSTERDAM, NL, vol. 254, no. 1-4, 28 avril 2006 (2006-04-28), pages 9-20, XP027903041, ISSN: 0044-8486 [extrait le 2006-04-28]
- DATABASE WPI Week 201079 Thomson Scientific, London, GB; AN 2010-K35903 XP002725849, & KR 2010 0088251 A (BEOM C Y) 9 août 2010 (2010-08-09)
- MARC LAHAYE ET AL: JOURNAL OF APPLIED PHYCOLOGY, vol. 11, no. 1, 1 janvier 1999 (1999-01-01), pages 1-7, XP055068568, ISSN: 0921-8971, DOI: 10.1023/A:1008063600071
- HUIMIN QI ET AL: "Antioxidant activity of different molecular weight sulfated polysaccharides from Ulva pertusa Kjellm (Chlorophyta)", JOURNAL OF APPLIED PHYCOLOGY, KLUWER ACADEMIC PUBLISHERS, DO, vol. 17, no. 6, 1 décembre 2005 (2005-12-01), pages 527-534, XP019247834, ISSN: 1573-5176

## Description

La présente invention concerne un extrait d'algues de l'ordre des ulvales, en particulier un extrait d'algues vertes de type *Ulva,* pour son utilisation pour moduler la réponse immunitaire chez un être humain ou un animal, en particulier pour stimuler la réponse immunitaire face à des infections. Elle concerne également l'utilisation non thérapeutique d'un extrait d'algues vertes de type *Ulva* pour moduler la réponse immunitaire chez un être humain ou animal.

La modulation de la réponse immunitaire est un axe de traitement important pour de nombreuses maladies. En effet, il est par exemple important d'améliorer la réponse immunitaire chez les patients souffrant d'infections.

La stimulation de la réponse immunitaire est par ailleurs un mécanisme important à bien des égards. On sait par exemple que le lait maternel est enrichi en IgAs, anticorps qui vont permettre la protection du nouveau né contre un certain nombre d'infections. Les cellules intestinales, en particulier ses cellules épithéliales, sont exposées à divers agents pathogènes contre lesquels des anticorps sont produits. Ces anticorps vont ensuite migrer jusqu'à la glande mammaire et se retrouver dans le lait maternel, procurant ainsi une protection au nourrisson. Il existe toujours un besoin d'identifier de nouvelles substances capables de stimuler cette réaction immunitaire. En particulier, les cellules épithéliales intestinales constituent un site privilégié au niveau duquel des substances immunostimulatrices administrées par voie orale pourraient agir. Il existe par ailleurs un besoin d'identifier de nouvelles substances capables d'augmenter la réponse à la vaccination, en stimulant la réponse immunitaire, en particulier via une administration complémentaire à la vaccination. Les espèces d'ulves (Ulvales, Chlorophyta) sont des algues abondantes trouvées dans la zone intertidale ou estran. Elles colonisent les substrats durs, ancrés par un disque de fixation, mais certaines espèces peuvent également donner lieu à des formes vivantes libres, à la dérive. Les ulves sont des algues à croissance rapide et opportunistes quant à l'espace et l'absorption des nutriments. Leur croissance dans la colonne d'eau est particulièrement observée dans les eaux côtières eutrophisées et dans les lagunes où *Ulva* sp. prolifère sous la forme de "marées vertes" (Fletcher, 1996). Il en résulte souvent une production de masse et des échouages massifs produisant des gaz nocifs lors de leur accumulation (Morand et Briand, 1996). Jusqu'à présent, cette biomasse a possédé une très faible valeur ajoutée et les moyens de l'utiliser en dehors du compost (Mazé et al. 1993, Cuomo et al. 1995), de la production de méthane (Briand et Morand, 1997), de la consommation alimentaire humaine (Pérez, 1997) ou comme base de papier (Nicolucci et Monegato 1993) pourraient permettre de profiter de leurs propriétés spécifiques. L'article "Stimulation of Turbot phagocytes by Ulva rigida C. Agardh polysaccharides" par R.Castro et al (Aquaculture 254 (2006) 9-20) est également mentionné ici.

Les inventeurs de la présente invention ont mis en évidence, de façon surprenante, qu'un extrait d'algues de l'ordre des ulvales, en particulier un extrait d'algues vertes de type *Ulva,* possédait des propriétés immunomodulatrices.

Ainsi, la présente invention concerne un extrait d'algues de l'ordre des ulvales, en particulier un extrait d'algues vertes de type *Ulva,* comprenant des polysaccharides polyanioniques sulfatés et non sulfatés dont la taille est inférieure ou égale à 50 kDa, (à l'exclusion de polysaccharides polyanioniques sulfatés et non sulfatés dont la taille discriminée par ultrafiltration est supérieure à 50 kDa) pour son utilisation pour moduler la réponse immunitaire chez un être humain ou un animal.

Elle concerne également l'utilisation non thérapeutique d'un extrait d'algues de l'ordre des ulvales, en particulier un extrait d'algues vertes de type *Ulva,* comprenant des polysaccharides polyanioniques sulfatés et non sulfatés dont la taille est inférieure ou égale à 50 kDa, pour moduler la réponse immunitaire chez un être humain ou animal.

En particulier, la présente invention concerne un extrait d'algues pour son utilisation telle que définie ci-dessus, ou l'utilisation non thérapeutique d'un extrait algues telle que définie ci-dessus, dans laquelle les polysaccharides comprennent du mannose et/ou de l'arabinose, préférentiellement du mannose. Plus particulièrement encore, lesdits polysaccharides comprennent au moins 0.005% de mannose et/ou au moins 0.005% d'arabinose, en poids par rapport au poids de la matière sèche totale de l'extrait d'algues, préférentiellement au moins 0.005% de mannose. Encore plus particulièrement, lesdits polysaccharides comprennent du mannose en une quantité allant de 0.005 à 0.5%, par exemple de 0.005 à 0.20% ou de 0.15 à 0.5% et/ou de l'arabinose en une quantité allant de 0.005 à 0.5%, en poids par rapport au poids de la matière sèche totale de l'extrait d'algues, préférentiellement du mannose en une quantité allant de 0.005 à 0.5%, par exemple de 0.005 à 0.20% ou de 0.15 à 0.5% en poids par rapport au poids de la matière sèche totale de l'extrait d'algues.

La présente invention concerne plus particulièrement encore un extrait d'algues pour son utilisation tel que définie ci-dessus, ou l'utilisation non thérapeutique d'un extrait algues telle que définie ci-dessus, dans laquelle lesdits polysaccharides comprennent en outre:
- du galactose; et/ou
- du glucose; et/ou
- du rhamnose; et/ou
- du xylose; et/ou
- de l'acide glucuronique.

Plus particulièrement, lesdits polysaccharides comprennent :
- de 0.05 à 0.5% de galactose; et/ou
- de 0.005 à 0.5% de glucose, en particulier de 0.005 à 0.05% ou de 0.05 à 0.5% ; et/ou;
- de 2 à 15 % de rhamnose; et/ou
- de 0.1 à 1% de xylose; et/ou
- de 1 à 7% d'acide glucuronique;
en poids par rapport au poids de la matière sèche totale de l'extrait d'algues. La présente invention concerne également un extrait d'algues de l'ordre des ulvales, en particulier extrait d'algues vertes de type *Ulva,* susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins; et
f) le jus de filtration obtenu à l'étape e) est concentré puis séché ;
pour son utilisation pour moduler la réponse immunitaire chez un être humain ou un animal.

Elle concerne également l'utilisation non thérapeutique d'un extrait d'algues de l'ordre des ulvales, en particulier d'un extrait d'algues vertes de type *Ulva,* susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins; et
f) le jus de filtration obtenu à l'étape e) est concentré puis séché ;
pour moduler la réponse immunitaire chez un être humain ou un animal.

En particulier, la présente invention concerne un extrait d'algues pour son utilisation telle que mentionnée ci-dessus, ou l'utilisation non thérapeutique d'un extrait d'algues telle qu'indiquée ci-dessus, pour stimuler la réponse immunitaire chez un être humain ou animal, en particulier au niveau du système immunitaire intestinal. Dans le cadre de la présente invention, on entend par propriétés « immunomodulatrices » ou permettant la « modulation de la réponse immunitaire », la signification habituellement apportée à ces termes et bien connue de l'homme du métier, en particulier toute propriété permettant de stimuler ou de freiner les réactions immunitaires du corps humain ou animal.

En particulier, l'extrait d'algues pour son utilisation selon l'invention, ou l'utilisation non thérapeutique de l'extrait d'algues selon l'invention, permet d'induire l'expression de molécules d'adhésion et de chimiokines, notamment les IL8 et/ou IL-1α et/ou IL1-β et/ou IL6 et/ou TNF- α et/ou CCL20.

Ainsi l'extrait d'algues tel que défini dans le cadre de la présente invention est utile dans le traitement et/ou la prévention des pathologies infectieuses, en particulier les pathologies infectieuses porcines choisies parmi : parvovirose, rouget, rhinite infectieuse, grippe (influenza), circovirose porcine, mycoplasmose et colibacillose ; les pathologies infectieuses aviaires choisies parmi : maladie de Marek, maladie de Newcastle, bronchite infectieuse, maladie de Gumboro, variole aviaire, mycoplasmose, anémie infectieuse aviaire, laryngotrachéite infectieuse, EDS et grippe aviaire ; les pathologies infectieuses bovines choisies parmi: BVD (diarrhées virales bovine), bronchopneumonie enzootique, IBR, herpes virose, clostridiose, colibacillose, fièvre catarrhale, coronavirose, rotavirose et rhinotrachéite ; et les pathologies infectieuses aquacoles choisies parmi: nécrose hématopoïétique, vibriose, furonculose, nécrose pancréatique infectieuse et anémie infectieuse.

La présente invention concerne donc également un extrait d'algues tel que décrit dans le cadre de la présente invention pour son utilisation dans le traitement et/ou la prévention d'une pathologie infectieuse telle que celles susmentionnées.

En particulier, l'extrait d'algues tel que défini dans le cadre de la présente invention est utile dans le cadre d'une prophylaxie vaccinale, en tant que complément à la vaccination.

Plus particulièrement, la présente invention concerne un extrait d'algues pour son utilisation telle qu'indiquée ci-dessus, dans laquelle l'extrait d'algues est utilisé dans une composition pharmaceutique pour une administration orale.

Selon un autre mode de réalisation de la présente invention, l'extrait d'algues pour son utilisation telle qu'indiquée ci-dessus, est utilisé dans une composition pharmaceutique par voie parentérale.

La présente invention concerne également l'utilisation non thérapeutique d'un extrait d'algues telle qu'indiquée ci-dessus, dans laquelle l'extrait d'algues est utilisé dans un complément alimentaire pour une administration orale.

Un extrait d'algues tel que décrit dans le cadre de la présente invention est notamment décrit dans la demande de brevet FR1261909.

Tel qu'indiqué ci-dessus, un extrait d'algues tel que décrit dans le cadre de la présente invention concerne un extrait d'algues de l'ordre des ulvales, en particulier un extrait d'algues vertes de type *Ulva,* comprenant des polysaccharides polyanioniques sulfatés et non sulfatés dont la taille est inférieure ou égale à 50 kDa. Plus particulièrement, l'extrait d'algues comprend des polysaccharides polyanioniques sulfatés et non sulfatés dont la taille est inférieure à 40, 30, 20 ou 15 kDa. Plus particulièrement encore, les polysaccharides polyanioniques sulfatés et non sulfatés de l'extrait d'algues ont une taille inférieure ou égale à 15 kDa.

L'extrait d'algues tel que décrit dans la cadre de la présente invention comprend des polysaccharides polyanioniques sulfatés et non sulfatés dont la taille est inférieure ou égale à 50 kDa, à l'exclusion de polysaccharides polyanioniques sulfatés et non sulfatés dont la taille est supérieure à 50 kDa.

Selon un autre mode réalisation de l'invention, l'extrait d'algues tel que décrit dans le cadre de la présente invention comprend des polysaccharides polyanioniques sulfatés et non sulfatés dont la taille est inférieure ou égale à 15 kDa, à l'exclusion de polysaccharides polyanioniques sulfatés et non sulfatés dont la taille est supérieure à 15 kDa.

Un dalton (Da) est une unité massique définie comme étant égal à un douzième de la masse d'un atome de carbone 12, masse qui s'avérera ensuite estimée à partir d'un mélange de plusieurs isotopes (principalement carbone 12 et carbone 13, possédant respectivement 6 et 7 neutrons en plus des 6 protons de tout atome de carbone). Un dalton est, avec une assez bonne précision, la masse d'un atome d'hydrogène, la valeur exacte étant 1,00794 uma (unité de masse atomique). Le kilodalton (kDa) est égal à 1000 Da.

Dans le cadre de la présente invention, les masses mentionnées en kDa sont déterminées par toute méthode usuellement employée par l'homme du métier, en particulier les masses des polysaccharides polyanioniques sulfatés et non sulfatés des extraits d'algues pourront être discriminées par ultrafiltration sur des membranes laissant uniquement filtrer des molécules de tailles prédéterminées.

En particulier, et tel que précédemment mentionné, les polysaccharides de l'extrait d'algues décrits dans le cadre de la présente invention comprennent du mannose et/ou de l'arabinose, de préférence du mannose. Plus particulièrement, lesdits polysaccharides comprennent au moins 0.005% de mannose et/ou au moins 0.005% d'arabinose, en poids par rapport au poids de la matière sèche totale de l'extrait d'algues, notamment au moins au 0.01 % de mannose et/ou au moins 0.01 % d'arabinose. Encore plus particulièrement, lesdits polysaccharides comprennent du mannose en une quantité allant de 0.01 à 0.50%, par exemple de 0.01 à 0.20% ou de 0.20 à 0.5% et/ou de l'arabinose en une quantité allant de 0.01 à 0.5%, en poids par rapport au poids de la matière sèche totale de l'extrait d'algues, notamment du mannose en une quantité allant de 0.03 à 0.45%, par exemple de 0.03 à 0.15% ou de 0.15 à 0.45% et/ou de l'arabinose en une quantité allant de 0.01 à 0.2%.

Préférentiellement, lesdits polysaccharides comprennent du mannose en une quantité allant de 0.01 à 0.50%, par exemple de 0.01 à 0.20% ou de 0.20 à 0.5%, notamment du mannose en une quantité allant de 0.03 à 0.45%, par exemple de 0.03 à 0.15% ou de 0.15 à 0.45%.

De façon particulière, et tel que mentionné précédemment, lesdits polysaccharides comprennent en outre:
- du galactose; et/ou
- du glucose; et/ou
- du rhamnose; et/ou
- du xylose; et/ou
- de l'acide glucuronique.

Plus particulièrement encore, lesdits polysaccharides comprennent :
- de 0.05 à 0.5% de galactose, notamment de 0.1 à 0.4%; et/ou
- de 0.005 à 0.5% de glucose, en particulier de 0.005 à 0.05%, notamment de 0.01 à 0.03%, ou de 0.05 à 0.5% ; %; et/ou
- de 2 à 15 % de rhamnose, notamment de 5 à 10%; et/ou
- de 0.1 à 1% de xylose, notamment de 0.3 à 0.7%; et/ou
- de 1 à 7% d'acide glucuronique, notamment de 1 à 5%;
en poids par rapport au poids de la matière sèche totale de l'extrait d'algues.

Ainsi, on peut par exemple citer un extrait d'algues pour son utilisation selon l'invention comprenant :
- du mannose ; et/ou
- de l'arabinose ; et/ou
- du galactose; et/ou
- du glucose; et/ou
- du rhamnose; et/ou
- du xylose; et/ou
- de l'acide glucuronique.
On peut plus particulièrement citer par exemple un extrait d'algues pour son utilisation selon l'invention comprenant :
- de 0.01 à 0.50% de mannose, par exemple de 0.01 à 0.20%, notamment de 0.03 à 0.15% ou de 0.20 à 0.5% ; et/ou
- de 0.01 à 0.5% d'arabinose, notamment de 0.01 à 0.2% ; et/ou
- de 0.05 à 0.5% de galactose, notamment de 0.1 à 0.4%; et/ou
- de 0.005 à 0.5% de glucose, en particulier de 0.005 à 0.05%, notamment de 0.01 à 0.03%, ou de 0.05 à 0.5%; et/ou
- de 2 à 15 % de rhamnose, notamment de 5 à 10%; et/ou
- de 0.1 à 1% de xylose, notamment de 0.3 à 0.7%; et/ou
- de 1 à 7% d'acide glucuronique, notamment de 1 à 5%;
en poids par rapport au poids de la matière sèche totale de l'extrait d'algues.
On peut encore plus particulièrement citer par exemple un extrait d'algues pour son utilisation selon l'invention comprenant :
- 0.09% de mannose; et/ou
- 0.1% d'arabinose ; et/ou
- 0.3% de galactose; et/ou
- 0.02% de glucose ; et/ou
- 8.1% de rhamnose; et/ou
- 0.5% de xylose; et/ou
- 2.6% d'acide glucuronique;
en poids par rapport au poids de la matière sèche totale de l'extrait d'algues.
On peut également citer par exemple un extrait d'algues pour son utilisation selon l'invention comprenant :
- 0.3% de mannose; et/ou
- 0.2% de galactose; et/ou
- 0.4% de glucose ; et/ou
- 7.9% de rhamnose; et/ou
- 0.5% de xylose; et/ou
- 4.9% d'acide glucuronique;
en poids par rapport au poids de la matière sèche totale de l'extrait d'algues.

La présente invention concerne donc également l'utilisation non thérapeutique d'un extrait d'algues selon l'invention, dans laquelle l'extrait d'algues comprend :
- du mannose ; et/ou
- de l'arabinose ; et/ou
- du galactose; et/ou
- du glucose; et/ou
- du rhamnose; et/ou
- du xylose; et/ou
- de l'acide glucuronique,
et plus particulièrement :
- de 0.01 à 0.50% de mannose, par exemple de 0.01 à 0.20%, notamment de 0.03 à 0.15% ou de 0.20à 0.5% ; et/ou
- de 0.01 à 0.5% d'arabinose, notamment de 0.01 à 0.2% ; et/ou
- de 0.05 à 0.5% de galactose, notamment de 0.1 à 0.4%; et/ou
- de 0.005 à 0.5% de glucose, en particulier de 0.005 à 0.05%, notamment de 0.01 à 0.03%, ou de 0.05 à 0.5% ; et/ou
- de 2 à 15 % de rhamnose, notamment de 5 à 10%; et/ou
- de 0.1 à 1% de xylose, notamment de 0.3 à 0.7%; et/ou
- de 1 à 7% d'acide glucuronique, notamment de 1 à 5%;
en poids par rapport au poids de la matière sèche totale de l'extrait d'algues,
et encore plus particulièrement comprenant :
- 0.09% de mannose; et/ou
- 0.1% d'arabinose ; et/ou
- 0.3% de galactose; et/ou
- 0.02% de glucose ; et/ou
- 8.1% de rhamnose; et/ou
- 0.5% de xylose; et/ou
- 2.6% d'acide glucuronique;
en poids par rapport au poids de la matière sèche totale de l'extrait d'algues,
ou comprenant :
- 0.3% de mannose; et/ou
- 0.2% de galactose; et/ou
- 0.4% de glucose ; et/ou
- 7.9% de rhamnose; et/ou
- 0.5% de xylose; et/ou
- 4.9% d'acide glucuronique;
en poids par rapport au poids de la matière sèche totale de l'extrait d'algues.

Tel qu'indiqué ci-dessus, l'extrait d'algues tel que décrit dans le cadre de la présente invention est un extrait d'algues de l'ordre des ulvales, en particulier un extrait d'algues vertes de type *Ulva,* susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins; et le jus de filtration obtenu à l'étape e) est concentré puis séché.

Selon un mode de réalisation de l'invention, l'extrait d'algues comprend :
- de 10 à 50 % de carbone ;
- de 1 à 10% d'hydrogène ;
- de 1 à 5 % d'azote ;
- de 20 à 50 % d'oxygène ; et
- de 1 à 15 % de soufre ;
en pourcentage massique de la matière sèche totale de l'extrait d'algues.
Plus particulièrement encore, l'extrait d'algues comprend :
- de 15 à 30 % de carbone ;
- de 3 à 6% d'hydrogène ;
- de 1 à 3 % d'azote ;
- de 25 à 40 % d'oxygène ; et
- de 2,5 à 10 % de soufre ;
en pourcentage massique de la matière sèche totale de l'extrait d'algues.
Selon un autre mode de réalisation de l'invention, l'extrait d'algues comprend :
- de 10 à 50 % de carbone ;
- de 1 à 10% d'hydrogène ;
- de 0,5 à 5 % d'azote ;
- de 20 à 60 % d'oxygène ; et
- de 1 à 15 % de soufre ;
en pourcentage massique de la matière sèche totale de l'extrait d'algues.
Les autres éléments chimiques présents dans la matière sèche de l'extrait sont notamment représentés par les minéraux (Ca, K, Na, Mg, Al, Cl, I, P, Fe, etc).

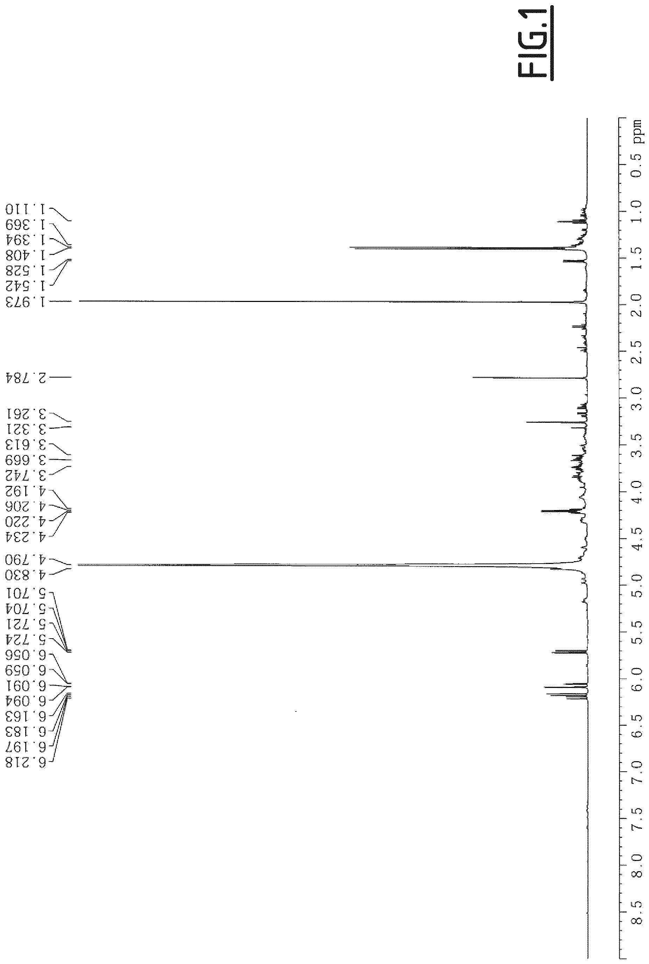

Plus particulièrement, l'extrait d'algues tel que décrit dans le cadre de la présente invention est caractérisé par le spectre RMN ¹H présenté en Figure 1.

Ce spectre RMN ¹H a été enregistré à 298 K sur un spectromètre Bruker Avance 500 équipé d'une sonde cryogénique inverse 5 mm ¹H/¹³C/¹⁵N TCl Avant l'analyse, les échantillons ont été dissous dans 99,97% d'atome de D₂O. Les déplacements chimiques sont exprimés en ppm par rapport à un étalon externe (acide triméthylsilylpropionique). Aucune suppression du signal HOD n'a été réalisée.

Selon un mode de réalisation de la présente invention, l'extrait d'algues comprenant en particulier des polysaccharides polyanioniques sulfatés et non sulfatés dont la taille est inférieure ou égale à 50 kDa est susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins ; et
f) le jus de filtration obtenu à l'étape e) est concentré puis séché.

En particulier, pour la mise en œuvre du procédé tel qu'indiqué dans le cadre de la présente invention, dans l'étape a) de celui-ci, les algues sont lavées à l'eau douce.

Elles peuvent être dessablées par tout moyen mis à disposition de l'homme du métier.

Lesdites algues sont ensuite broyées, notamment au moyen d'un broyeur, comme par exemple un affineur ou un cutteur.

Par la suite, la phase solide du broyat, le marc, est séparée de sa phase liquide, le jus, par pressage du broyat, par exemple à l'aide d'une presse à bande ou à plateaux, ou par centrifugation.

Par « jus », on entend le jus cytoplasmique qui inclue la structure pariétale de la double structure des cellules des algues.

La phase liquide obtenue est ensuite clarifiée, par exemple avec un clarificateur à assiettes, ou par centrifugation, décantation ou filtration (par exemple à poche ou à plaque).

Le jus obtenu est ensuite ultrafiltré.

Selon un mode de réalisation pour la mise en œuvre du procédé tel qu'indiqué dans le cadre de la présente invention, l'ultrafiltration est réalisée sur une membrane de 50 kDa ou moins, notamment sur une membrane de 40, 30, 20 ou 15 kDa. Plus particulièrement, la membrane sera une membrane de 15 kDa ou moins.

Cette membrane peut être par exemple une membrane de céramique ou une membrane organique. Plus particulièrement, la membrane est une membrane de céramique.

Le jus de filtration obtenu peut ensuite être concentré, par exemple par osmose inverse, évaporation ou précipitation, puis séché par exemple par lyophilisation ou atomisation.

Optionnellement, l'extrait obtenu pourra ensuite être broyé de nouveau, afin d'obtenir une poudre homogène en terme de granulométrie.

Selon un de ces aspects, le procédé se déroule en partie à température ambiante. Par température ambiante, on entend une température comprise entre 5 et 25°C.

Selon un autre de ces aspects, le procédé se déroule en partie à une température comprise entre 4 et 10°C, ceci afin d'éviter les développements microbiens.

Selon un mode de réalisation pour la mise en œuvre du procédé tel qu'indiqué dans le cadre de la présente invention, l'extrait d'algues obtenu à l'étape f) du procédé susmentionné est purifié, par exemple par ultrafiltration, en particulier sur une cassette d'ultrafiltration, notamment afin d'éliminer la partie minérale.

Selon un autre de ces aspects, l'extrait d'algues pour son utilisation selon l'invention est obtenu par le procédé tel que précédemment décrit.

Ce procédé diffère de la plupart des procédés décrits dans l'art antérieur du fait de l'absence d'une étape impliquant une précipitation de l'extrait d'algues. Il se distingue également des précédents procédés d'extraction de par l'absence d'utilisation de solvants, en particulier organiques, ce qui représente un atout majeur du point de vue écologique.

Par « algues vertes de type *Ulva* » on entend les algues vertes regroupées dans le genre *Ulva,* de la famille des Ulvaceae, de l'ordre des ulvales. On peut notamment citer les espèces et sous-espèces suivantes : *Ulva acanthophora, Ulva anandii, Ulva angusta, Ulva arasakii, Ulva armoricana, Ulva atroviridis, Ulva attenuata, Ulva beytensis, Ulva bifrons, Ulva brevistipitata, Ulva bulbosa, Ulva burmanica, Ulva byssoides, Ulva californica, Ulva chaetomorphoides, Ulva clathrata, Ulva coccinea, Ulva compressa, Ulva conglobata, Ulva cornucopiae, Ulva cornuta, Ulva covelongensis, Ulva crassa, Ulva crassimembrana, Ulva curvata, Ulva dactylifera, Ulva denticulata, Ulva elegans, Ulva elminthoides, Ulva enteromorpha, Ulva erecta, Ulva expansa, Ulva fasciata, Ulva fenestrata, Ulva flexuosa, Ulva gelatinosa, Ulva geminoidea, Ulva gigantea, Ulva grandis, Ulva hendayensis, Ulva hookeriana, Ulva hopkirkii, Ulva indica, Ulva intestinalis, Ulva intestinaloides, Ulva intricata, Ulva intybacea, Ulva javanica, Ulva kylinii, Ulva lactuca, Ulva lactucaefolia, Ulva laetevirens, Ulva laingii, Ulva linearis, Ulva lingulata, Ulva linkiana, Ulva linza, Ulva lippii, Ulva litoralis, Ulva littorea, Ulva lobata, Ulva lubrica, Ulva marginata, Ulva micrococca, Ulva myriotrema, Ulva neapolitana, Ulva nematoidea, Ulva ohnoi, Ulva olivacea, Ulva olivaceum, Ulva pacifica, Ulva papenfussii, Ulva paradoxa, Ulva parva, Ulva parvula, Ulva patengensis, Ulva percursa, Ulva pertusa, Ulva phyllosa, Ulva popenguinensis, Ulva porrifolia, Ulva procera, Ulva profunda, Ulva prolifera, Ulva pseudocurvata, Ulva pseudolinza, Ulva pulchra, Ulva purpurascens, Ulva quilonensis, Ulva radiata, Ulva ralfsii, Ulva ranunculata, Ulva reticulata, Ulva rhacodes, Ulva rigida, Ulva rotundata, Ulva rubens, Ulva saifullahii, Ulva scagelii, Ulva scandinavica, Ulva sericea, Ulva serrata, Ulva simplex, Ulva sorensenii, Ulva spinulosa, Ulva stenophylla, Ulva stipitata, Ulva sublittoralis, Ulva subulata, Ulva taeniata, Ulva tenera, Ulva tetragona, Ulva torta, Ulva tuberosa, Ulva umbilicata, Ulva uncialis, Ulva uncinata, Ulva usneoides, Ulva utricularis, Ulva utriculosa, Ulva uvoides, Ulva ventricosa.*

Ainsi, un extrait d'algues pour son utilisation selon la présente invention peut être utilisé dans des applications vétérinaires, et être compris dans un médicament ou une composition pharmaceutique, pour moduler la réponse immunitaire chez un être humain ou un animal, en particulier pour stimuler la réponse immunitaire chez un être humain ou animal, et plus particulièrement pour prévenir et/ou traiter une pathologie infectieuse telle que celles susmentionnées, encore plus particulièrement dans le cadre d'une prophylaxie vaccinale, en tant que complément à la vaccination.

Ainsi la présente invention concerne également un extrait d'algues pour son utilisation telle que précédemment mentionnée, dans laquelle l'extrait d'algues est compris dans une composition pharmaceutique ou dans un médicament.

L'utilisation non thérapeutique d'un extrait d'algues selon la présente invention peut quant à elle cibler des applications destinées à l'être humain ou à l'animal, par exemple par le biais de compléments alimentaires à visée santé sans effets secondaires pour moduler la réponse immunitaire chez un être humain ou un animal, en particulier pour stimuler la réponse immunitaire chez un être humain ou animal.

Ainsi la présente invention concerne également l'utilisation non thérapeutique d'un extrait d'algues telle que précédemment mentionnée, dans laquelle l'extrait d'algues est compris dans une composition alimentaire.

En particulier, la composition alimentaire est utile en tant que complément à la vaccination.

Les excipients pharmaceutiquement acceptables utilisés pour la préparation d'un médicament ou d'une composition pharmaceutique comprenant un extrait d'algues pour son utilisation selon l'invention sont choisis selon la forme pharmaceutique et le mode d'administration souhaité, parmi les excipients habituels qui sont connus de l'homme du métier.

Dans la présente invention pour l'administration orale, sublinguale, sous-cutanée, intramusculaire, intra-veineuse, topique, locale, intratrachéale, intranasale, transdermique ou rectale, l'extrait d'algues tel que défini ci-dessus, peut être administré sous forme de doses unitaires, en mélange avec des excipients pharmaceutiques classiques, aux animaux et aux êtres humains, pour moduler la réponse immunitaire, en particulier pour prévenir et/ou le traiter des pathologies infectieuses telles que susmentionnées.

Les formes d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, sous cutanée, intramusculaire ou intraveineuse, les formes d'administration rectale et les implants. Pour l'application topique, on peut utiliser les extraits d'algues pour leur utilisation selon l'invention dans des crèmes, gels, pommades ou lotions.

Lorsqu'une composition solide sous forme de comprimés est préparée, l'ingrédient actif principal peut être mélangé avec un véhicule pharmaceutique, tel que la gélatine, l'amidon, le lactose, le stéarate de magnésium, le talc, la gomme arabique ou analogues.

Les comprimés peuvent également être enrobés avec du saccharose, un dérivé cellulosique, ou d'autres matières appropriées ou encore ils peuvent être traités de telle sorte qu'ils aient une activité prolongée ou retardée et qu'ils libèrent d'une façon continue une quantité prédéterminée de principe actif.

Une préparation sous forme de gélules peut par exemple être obtenue en mélangeant l'ingrédient actif avec un diluant et en versant le mélange obtenu dans des gélules molles ou dures.

Dans un mode de réalisation particulier, l'extrait d'algues, le médicament ou la composition pharmaceutique pour son utilisation selon la présente invention est destiné à une administration orale. Dans un autre mode de réalisation, l'extrait d'algues, le médicament ou la composition pharmaceutique pour son utilisation selon la présente invention est destiné à une administration parentérale.

Les médicaments ou compositions pharmaceutiques comprenant un extrait d'algues pour son utilisation selon l'invention, peuvent aussi se présenter sous forme liquide, par exemple, sous forme de solutions, d'émulsions, de suspensions ou de sirops, et notamment sous une forme adaptée pour une administration orale ou intranasale, par exemple. Les supports liquides appropriés peuvent être, par exemple, de l'eau, des solvants organiques tels que le glycérol ou les glycols, de même que leurs mélanges, dans des proportions variées, dans l'eau.

Une préparation sous forme de sirop ou d'élixir ou pour l'administration sous forme de gouttes peut également contenir l'ingrédient actif conjointement avec un édulcorant, acalorique par exemple, du méthylparabène et du propylparabène comme antiseptique, ainsi qu'un agent donnant du goût et un colorant approprié.

Les poudres ou les granules dispersibles dans l'eau peuvent par exemple contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

En général, dans le cadre de la présente invention, la dose journalière de l'extrait d'algues sera la dose efficace la plus faible de l'extrait d'algues capable de produire un effet immunostimulateur.

Par « dose efficace », on désigne toute quantité d'une composition qui permet d'observer l'effet recherché, ici un effet immunostimulateur.

Selon un de ses aspects, un extrait d'algues pour son utilisation selon l'invention est utilisé dans une composition telle que susmentionnée pour une administration à une dose chez l'humain comprise entre 0,1 et 100 mg/kg, encore plus particulièrement entre 0,5 et 60 mg/kg, par exemple entre 1 et 20 mg/kg ou entre 5 et 30 mg/kg, ou à une dose chez l'animal comprise entre 1 et 200 mg/kg, plus particulièrement entre 1 et 100 mg/kg, encore plus particulièrement entre 2 et 45 mg/kg ou entre 10 et 60 mg/kg.

Selon un autre de ses aspects, dans le cadre de l'utilisation non thérapeutique d'un extrait d'algues selon la présente invention, celui-ci peut être utilisé dans une composition alimentaire.

Par « composition alimentaire », on entend par exemple tout type d'alicament, de produits alimentaires sous la forme de yaourt ou breuvage, lacté notamment, tout type de matière première, additif ou auxiliaire technologique, sous forme de prémélanges, médicamenteux ou non, destinés à être incorporés dans des aliments, tout type d'aliments complets ou complémentaires, destinés à l'homme ou l'animal.
Selon un de ses aspects, dans le cadre de l'utilisation non thérapeutique d'un extrait d'algues dans une composition alimentaire, celui-ci est utilisé pour une administration à une dose chez l'humain comprise entre 0,1 et 100 mg/kg, encore plus particulièrement entre 0,5 et 60mg/kg, par exemple entre 1 et 20 mg/kg ou entre 5 et 30 mg/kg, ou à une dose chez l'animal comprise entre 1 et 200 mg/kg, plus particulièrement entre 1 et 100 mg/kg, encore plus particulièrement entre 2 et 45 mg/kg ou entre 10 et 60 mg/kg.

La présente invention, selon un autre de ces aspects, concerne également une méthode pour moduler la réponse immunitaire chez un être humain ou un animal, en particulier pour stimuler la réponse immunitaire chez un être humain ou un animal qui comprend l'administration, à un être humain ou un animal, d'une dose efficace d'un extrait d'algues selon l'invention.

Plus particulièrement la méthode selon la présente invention stimule la réponse immunitaire chez un être humain ou un animal, en particulier au niveau du système immunitaire intestinal.

Plus particulièrement encore, la méthode selon l'invention induit l'expression de molécules d'adhésion et de chimiokines, notamment les IL8 et/ou IL-1α et/ou IL1-β et/ou IL6 et/ou TNF- α et/ou CCL20.

L'extrait d'algues administré dans le cadre de la méthode selon l'invention peut être compris dans un médicament, une composition pharmaceutique ou une composition alimentaire, tel que susmentionné.

Il peut être administré selon les modes d'administration susmentionnés.

Selon un mode de réalisation pour la mise en œuvre de la méthode selon l'invention, l'extrait d'algues selon l'invention est administré dans une composition pharmaceutique.

En particulier, ladite méthode est utile pour la prévention et/ou le traitement d'une pathologie infectieuse telle que celles susmentionnées.

Selon un autre mode de réalisation pour la mise en œuvre de la méthode selon l'invention, l'extrait d'algues selon l'invention est administré dans une composition alimentaire.

En particulier, la présente invention concerne également une méthode telle que susmentionnée qui comprend l'administration, à un être humain ou un animal, d'une dose efficace d'un extrait d'algues selon l'invention pendant une période de 3 à 30 jours, en particulier de 10 à 20 jours ou de 3 à 10 jours, à une dose chez l'humain comprise entre 0,1 et 100 mg/kg, plus particulièrement entre 0,5 et 60 mg/kg, encore plus particulièrement entre 1 et 20 mg/kg ou entre 5 et 30 mg/kg, ou à une dose chez l'animal comprise entre 1 et 200 mg/kg, plus particulièrement entre 1 et 100 mg/kg, encore plus particulièrement entre 2 et 45 mg/kg ou entre 10 et 60 mg/kg.

Selon un mode de réalisation pour la mise en œuvre de la méthode selon l'invention, à l'issu de la période de temps susmentionnée, l'administration de l'extrait d'algues peut être renouvelée pour une période équivalente.

Selon un de ces aspects, la présente invention concerne également une méthode telle que susmentionnée qui comprend :
a) la préparation d'un extrait d'algues selon l'invention par le procédé suivant :
   - lavage et dessablage des algues ;
   - broyage desdites algues;
   - séparation de la phase solide du broyat de sa phase liquide ;
   - clarification de ladite phase liquide;
   - ultrafiltration du jus obtenu à l'étape précédente sur une membrane de 50 kDa ou moins, par exemple de 15 kDa ou moins; et
   - concentration puis séchage du jus de filtration obtenu à l'étape précédente ; suivie optionnellement d'une étape d'ultrafiltration, par exemple sur une cassette d'ultrafiltration ; et
b) l'administration, à un être humain ou un animal, d'une dose efficace d'un extrait d'algues selon l'invention, en particulier pendant une période de 3 à 30 jours, plus particulièrement de 10 à 20 jours ou de 3 à 10 jours, à une dose chez l'humain comprise entre 0,1 et 100 mg/kg, encore plus particulièrement entre 0,5 et 60 mg/kg, par exemple entre 1 et 20 mg/kg ou entre 5 et 30 mg/kg, ou à une dose chez l'animal comprise entre 1 et 200 mg/kg, plus particulièrement entre 1 et 100 mg/kg, encore plus particulièrement entre 2 et 45 mg/kg ou entre 10 et 60 mg/kg.

La présente invention sera illustrée plus en détail par les figures et exemples ci-dessous qui n'en limitent pas la portée.

### FIGURES

**Figure 1** : Spectre RMN ¹H d'un extrait d'algues selon la présente invention
**Figure 2** : Chromatogramme obtenu avec un extrait d'algues selon l'invention séparé sur deux colonnes shodex 802 et 803
**Figure 3** **:** Chromatogramme obtenu après l'analyse de dérivés triméthylsilylés de l'échantillon d'un extrait d'algues selon l'invention par chromatographie en phase gazeuse. Avec Ara: Arabinose; Gal: Galactose; Glc: Glucose ; Xyl: Xylose; Man: Mannose; Rha: Rhamnose, GlcA : Acide Glucuronique
**Figure 4** **:** Evaluation de la dose maximale non toxique de l'extrait d'algues EA1 selon l'invention
**Figure 5** : Evaluation de la dose maximale non toxique de l'extrait d'algues EA2 selon l'invention
**Figure 6** : Evaluation de la dose maximale non toxique de l'extrait d'algues EA3 selon l'invention
**Figure 7** : Effets des extraits d'algues EA1, EA2 et EA3 selon l'invention sur l'expression d'IL8 (mesure par qPCR)
**Figure 8** : Effets des extraits d'algues EA1, EA2 et EA3 selon l'invention sur l'expression de TNF-α (mesure par qPCR)
**Figure 9** : Effets des extraits d'algues EA1, EA2 et EA3 selon l'invention sur l'expression de CCL20 (mesure par qPCR)
**Figure 10** : Effets de l'extrait d'algues EA1 selon l'invention sur l'expression d'IL6 (mesure par qPCR)
**Figure 11** : Effets de l'extrait d'algues EA1 selon l'invention sur l'expression d'IL1 α (mesure par qPCR)
**Figure 12** : Effets de l'extrait d'algues EA1 selon l'invention sur l'expression d'IL1 β (mesure par qPCR)
**Figure 13** : Effets de l'extrait d'algues EA1 selon l'invention sur l'expression de PPARγ (mesure par qPCR)
**Figure 14** : Effets de l'extrait d'algues EA1 selon l'invention sur l'expression d'IL12p35 (mesure par qPCR)
**Figure 15** : Effets des extraits d'algues EA1 et EA3 selon l'invention mesuré par ELISA sur l'expression de TNF-α
**Figure 16** : Effets des extraits d'algues EA1 et EA3 selon l'invention mesuré par ELISA sur l'expression d'IL-8

### EXEMPLES

### Exemple 1 : Préparation d'un extrait d'algues selon l'invention

Une tonne d'algues vertes de type *Ulva,* fraîches, brutes, sont lavées à l'eau douce et dessablées à l'aide d'une machine à laver les algues.

Sauf indications contraires, les étapes du procédé sont réalisées à température ambiante.

Les algues (1 tonne d'algues égouttées à 8% de matière sèche) sont ensuite broyées en fines particules au moyen d'un affineur industriel (marque Inotec type "I175CDI-75D"). Par «fines particules », on entend des particules dont la taille est comprise entre 50 et 1000 nm, avec deux populations, la première dont les tailles sont comprises entre 50 et 200 nm, la seconde dont les tailles sont comprises entre 600 et 1000 nm.

Le broyat est ensuite pressé au moyen d'une presse à bande industrielle de marque Flottweg type "B FRU 800 HK" à un débit d'environ 1 tonne/heure.

Cette étape permet la séparation de la phase solide (marc) de la phase liquide (jus). Le rendement en jus obtenu est de 75%.

Les 750 kg de jus brut obtenus sont ensuite clarifiés au moyen d'un clarificateur à assiettes de marque Flottweg type "AC 2000".

On obtient ainsi 710 kg d'un jus clair à 3.10 % de masse sèche (95 à 98% de rendement en masse) et une crème (2 à 5% en masse).

Par la suite, le jus clair est ultrafiltré sur une membrane céramique (Tami Industries) de 15 KDa.

On obtient ainsi un perméat et un rétentat. Le perméat est conservé jusqu'à l'obtention de 640 kg de jus de filtration (91% de rendement en volume) à 2.2% de matière sèche.

Le jus de filtration (perméat) est alors séché par lyophilisation après concentration par évaporation.

La concentration est réalisée sur un évaporateur simple effet (EVA 1000, Pignat) avec les paramètres suivants: recirculation forcée, débit d'alimentation 10L/h, pression vapeur de 1 bar, pression de vide de 0,3 bar et température d'évaporation de 90°C.

Une première concentration est réalisée avec un débit d'eau évaporée de 8 L/h et le °brix monte de 5.5 (égal à une concentration de matière sèche de 4.5%) à 14.7.

Cette solution est ensuite concentrée une deuxième fois avec un débit d'eau évaporée de 5-6 L/h et le °brix monte jusqu'à 34. La concentration de matière sèche de la solution est déterminée à 38.4%.

La lyophilisation est ensuite réalisée à l'aide d'un appareil Bioblock scientific (modèle CHRIST alpha 1-4 LSC) à une température de congélation de -80°C qui est également la température minimale au cours de cette étape.

La poudre obtenue est ensuite broyée avec un broyeur planétaire MiniMill de marque Philips. Le produit a été introduit dans des bols de broyage (10 g de produit dans chaque bol de broyage avec 4 billes en zircone). L'ensemble a été mis en rotation pendant 15 minutes à la vitesse 10.

On obtient ainsi 14 kg de poudre d'extrait d'algues.

### Exemple 2 : Détermination de la taille des polysaccharides polyanioniques sulfatés et non sulfatés d'un extrait d'algues selon l'invention par GPC (Gel Permeation Chromatography)

L'extrait d'algues selon l'invention et préparé suivant l'exemple 1 est ultrafiltré sur une membrane de 1000 Da et est dissous à une concentration de 0.5 g/L dans de l'eau. Il est ensuite injecté sur deux colonnes shodex 802 et 803 placées en série (domaine de fractionnement de la colonne 802 : 4.10³ Da et de la colonne 803: 1.7.10⁵ Da). L'éluant utilisé est le nitrate de sodium 0.1 M avec de l'azoture de sodium 0.2% à un débit de 0.5 ml/min. La détection est réalisée via un réfractométre Wyatt et un détecteur à diffusion de la lumière 18 angles Wyatt. Les dn/dc sont pris égaux à 0.150 mL/g.

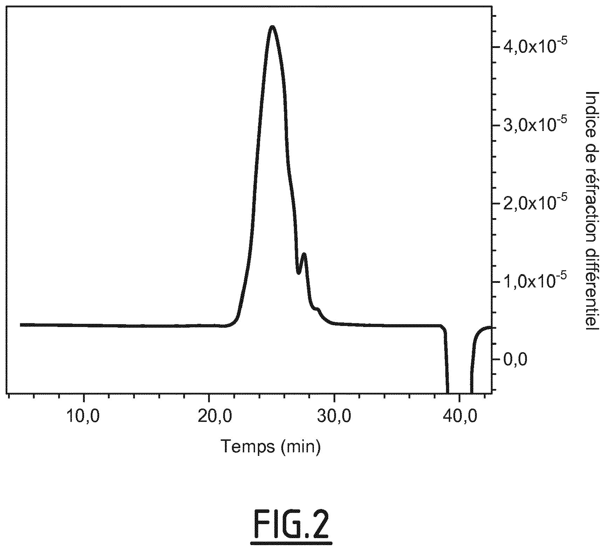

Le chromatogramme détecté par le refractomètre est présenté en figure 2.

On obtient une taille moyenne des polysaccharides d'un extrait d'algues selon l'invention_de 4.4 KDa.

### Exemple 3 : Détermination de la composition d'un extrait d'algues selon l'invention

L'extrait d'algues selon l'invention et préparé selon l'exemple 1 est purifié par ultrafiltration frontale dans des cellules amicon fonctionnant sous agitation. Une membrane en cellulose régénérée de seuil de coupure de 1000 Da est utilisée. 572.1 mg d'échantillon de l'extrait d'algues selon l'invention sont dissous dans 150 ml d'eau ultrapure milli-Q. Cinq litres d'eau sont utilisés pour éliminer toutes les molécules de masse inférieure à 1000 Da. Le rétentat est lyophilisé. 117 mg d'échantillon sont pesés. Le rendement de l'ultrafiltration est donc de 20.5% (p/p). Les analyses suivantes ont été effectuées sur les échantillons ultrafiltrés.

Le ratio des monosaccharides constitutifs des polysaccharides de l'extrait d'algues selon l'invention est déterminé selon la méthode de Kamerling (Kamerling *et al.,* 1975) modifiée par Montreuil (Montreuil *et al.,* 1986). L'identification et le dosage des monosaccharides nécessitent une hydrolyse par méthanolyse du polymère, de façon à n'obtenir que des monomères. Les résidus glycosidiques sont ensuite triméthylsilylés afin de les rendre volatils. Ils sont ainsi identifiés et dosés par chromatographie en phase gazeuse sous forme de méthylglycosides Otriméthylsilylés.

Les réactifs suivants sont utilisés :
- Solution de méthanol/HCl 3N (Supelco) ;
- Carbonate d'argent;
- Myo-inositol ;
- Pyridine ;
- Réactif Sylon BFT (BSTFA +TMCS 99 :1) (Supelco) ; et
- Dichlorométhane.

Le mode opératoire est le suivant : 400 µg de l'extrait d'algues selon l'invention préparé tel que mentionné ci-dessus et 50 µg de *myo-*inositol sont placés dans un bain à sec en présence de 500 µl d'un mélange méthanol/acide chlorhydrique 3 N (Supelco) pendant 4 heures à 100°C. Après refroidissement à température ambiante, le méthanolysat est neutralisé à l'aide de carbonate d'argent. Les échantillons sont centrifugés 15 minutes à 3000 tr/min et le surnageant est évaporé sous jet d'azote. Les composés sont ensuite dissous dans 80 µl de pyridine et incubés durant 25 minutes à 80°C avec 80 µl de sylon (BSTFA : TMCS, 99:1, Supelco). Après évaporation douce des réactifs en excès sous jet d'azote, les méthylglycosides triméthylsilylés sont repris dans 500 µl de dichlorométhane puis injectés en chromatographie en phase gazeuse (injection *in*-colonne, détecteur FID : ionisation de flamme). Le gaz vecteur est l'azote. La colonne, de type HP-5MS (30 m, 0.25 mm de diamètre interne), est apolaire. Le programme de montée en température est le suivant: 120°C maintenus pendant 1 minute, puis un gradient de 1.5°C/min jusqu'à 180°C, suivi d'un gradient de 2°C/min jusqu'à 200°C.

Chaque monosaccharide est identifié par comparaison de ses temps de rétention relatifs par rapport à l'étalon interne, avec ceux des monosaccharides purs traités dans les mêmes conditions. Un coefficient de réponse est calculé pour chaque monosaccharide par rapport à l'étalon interne afin de définir la proportion de chaque monosaccharide au sein des polysaccharides de l'extrait d'algues selon l'invention.

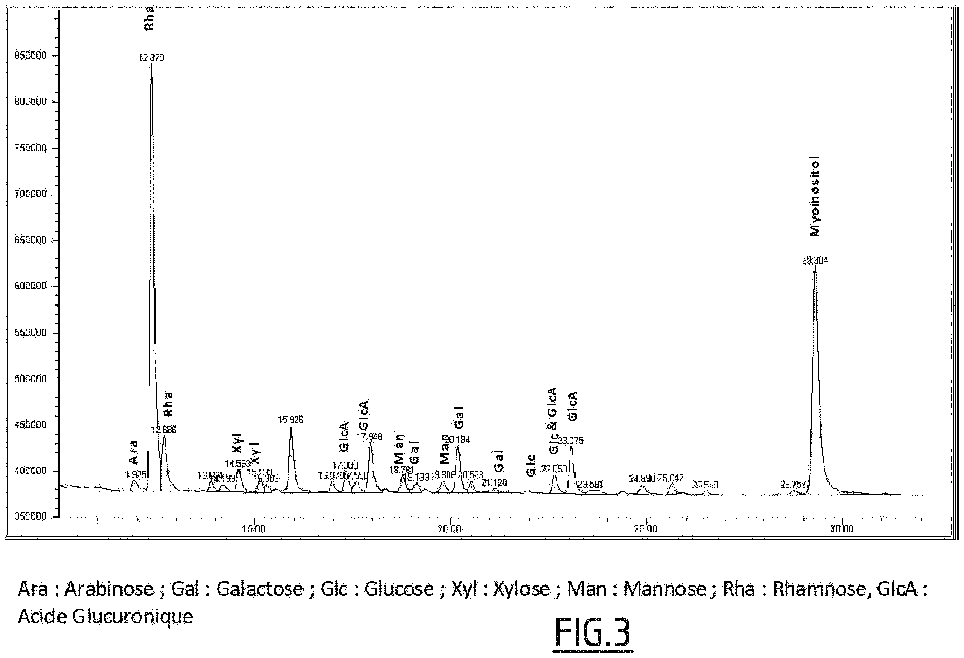

Les résultats obtenus sont présentés dans le tableau 1 ci-dessous et en figure 3.

**Tableau 1 : Composition de l'extrait d'algues selon l'invention obtenue après l'analyse de dérivés triméthylsilylés par chromatographie en phase gazeuse, exprimée en poids par rapport au poids total de l'extrait d'algues; avec Ara: Arabinose ; Gal: Galactose ; Glc: Glucose ; Xyl: Xylose ; Man: Mannose ; Rha: Rhamnose, GlcA: Acide Glucuronique.**

| Echantillon | % en poids de l'ultrafiltrat | Rendement ultrafiltration | % en poids de l'extrait brut |
|---|---|---|---|
| Ara | 0,6 | 20,50% | 0,123 |
| Gal | 1,3 | 20,50% | 0,267 |
| Glc | 0,1 | 20,50% | 0,021 |
| Xyl | 2,45 | 20,50% | 0,502 |
| Man | 0,45 | 20,50% | 0,092 |
| Rha | 39,6 | 20,50% | 8,118 |
| GlcA | 12,9 | 20,50% | 2,645 |

### Exemple 4 : Evaluation de l'activité de l'extrait d'algues selon l'invention pour son activité immunomodulatrice

Les effets de l'extrait d'algues selon l'invention sont testés sur des cellules épithéliales porcines différenciées IPEC-1.

Les cellules épithéliales intestinales porcines (Intestinal Porcine Epithelial Cell-1, IPEC-1) sont des cellules jéjunales de porcelet spontanément transformées.

Au préalable, la quantité optimale de cellules IPEC-1 à déposer dans les puits d'une flasque P6 pour atteindre la confluence en 3 jours est déterminée. La dose de 0,2 10⁵ cellules/cm² est retenue comme étant cette dose optimale.

La dose maximale non toxique de l'extrait d'algues selon l'invention est ensuite étudiée afin de déterminer la dose maximale qui n'inhibe pas la prolifération cellulaire des cellules IPEC-1.
Deux extraits d'algues selon l'invention sont préparés suivant l'exemple 1 (respectivement nommés EA1 et EA2).
Un troisième extrait d'algues EA3, également préparé suivant l'exemple 1, mais ayant été soumis à une étape de purification ultérieure est préparé.
Pour la purification, l'extrait EA3 est ultrafiltré sur une cassette PALL minimate de seuil de coupure 1KDa. Cet extrait est ensuite lyophilisé.

Les trois extraits sont dissous dans 28 mL de milieu DMEM/F12 complet. 50 mL de solution à 1% sont ensuite préparés, soit 0,5 g repris dans 50 mL. Une stérilisation par filtration à 0,2 µm est ensuite effectuée.
Des dilutions en tube Falcon 50 mL sont ensuite réalisées pour tester différentes concentrations d'extrait d'algues, comme indiqué dans le tableau 2 ci-dessous.

**Tableau 2**

| **Préparation solution extrait d'algues** | | | | | |
|---|---|---|---|---|---|
| | 1 % | 0,5 % | 0,1 % | 0,05 % | 0,01 % |
| dilution désirée | | 2 | 5 | 2 | 5 |
| dilution obtenue | | 2 | 5 | 2 | 5 |
| volume dilution précédente | | 19 | 9,5 | 18 | 6 |
| volume milieu | 48 | 19 | 37,8 | 18 | 24 |
| volume total | 48 | 38 | 47,3 | 36 | 30 |
| reste | 29,0 | 28,5 | 29,3 | 30,0 | 30,0 |

Les cellules sont ensuite préparées en suivant le protocole suivant :
1. préparation de 28 ml de suspension cellulaire à 0,07 10⁶ cellules/mL pour chaque dose à tester soit 1,96 10⁶ cellules/dose,
2. trypsinisation,
3. les cellules sont rincées avec du PBS sans Ca-Mg,
4. dépôt de Trypsine ATV (2 mL/F175, ou 0,4 mL/puits P6) pour dissocier et décoller les cellules,
5. incubation 5 minutes à 37°C,
6. reprise en milieu de culture,
7. numération au bleu trypan,
8. préparation de 6 tubes Falcon de 15 mL avec 2,23 mL,
9. centrifugation, et
10. reprise du culot de cellules de chaque tube dans 28 mL de chaque solution d'extrait à tester et transfert dans un tube Falcon de 50 mL.
La mise en culture est effectuée en déposant 3 ml/puits de chaque suspension dans les plaques puis en incubant à 37°C pendant 24, 48 et 72h.
Le tableau 3 résume le contenu des différents puits.

**Tableau 3**

| **Dose** | **Volume** | **Qté extrait** | **Nb cellules** | **Qté extrait/cellule** | | |
|---|---|---|---|---|---|---|
| **g/100ml** | **ml/puits** | **mg/puits** | **par puits** | **mg** | **µg** | **ng** |
| 0 | 3 | 0 | 2,1 E+05 | 0 | 0,0000 | 0,0 |
| 0,01 | 3 | 0,3 | 2,1 E+05 | 1,4E-06 | 0,0014 | 1,4 |
| 0,05 | 3 | 1,5 | 2,1 E+05 | 7,1 E-06 | 0,0071 | 7,1 |
| 0,1 | 3 | 3 | 2,1E+05 | 1,4E-05 | 0,0143 | 14,3 |
| 0,5 | 3 | 15 | 2,1 E+05 | 7,1 E-05 | 0,0714 | 71,4 |
| 1 | 3 | 30 | 2,1 E+05 | 1,4E-04 | 0,1429 | 142,9 |

Les cultures sont ensuite observées et des photos sont éventuellement prises.
Les cellules sont récoltées et énumérées suivant le protocole suivant :
1. prendre un flasque 6 puits,
2. retirer le surnageant,
3. rincer avec 1 mL de PBS sans Ca-Mg,
4. déposer 0,4 mL/puits de trypsine ATV,
5. incuber 5 min à 37°C jusqu'au décollement des cellules,
6. ajouter 0,6 ml de milieu de culture,
7. bien mélanger, et
8. utiliser Bleu Trypan 0,4%, (50µL de cellules + 50 µL de Bleu Trypan 0,4%), puis compter les cellules sur hématimètre de Malassez sur un total de 100 rectangles.

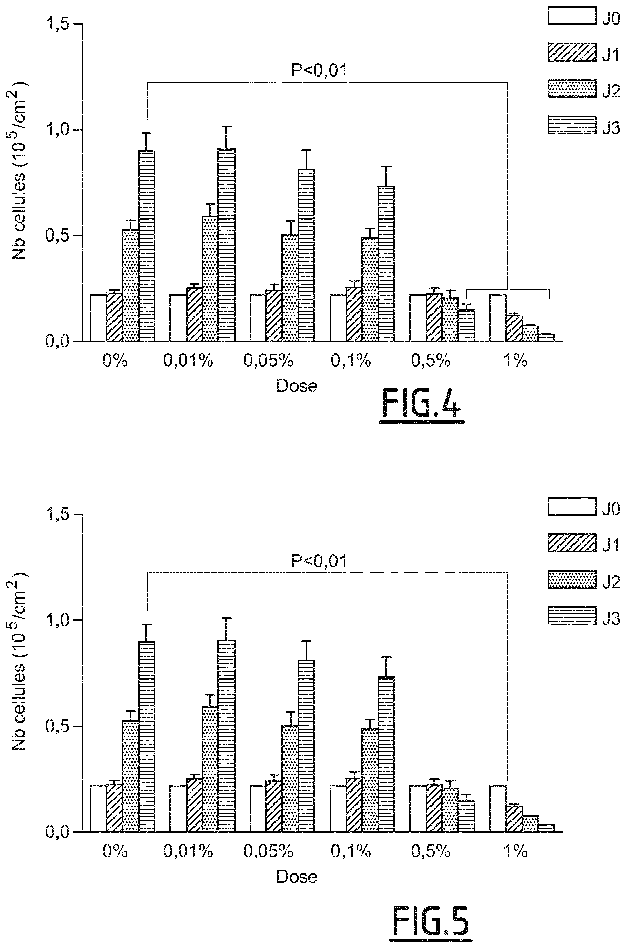

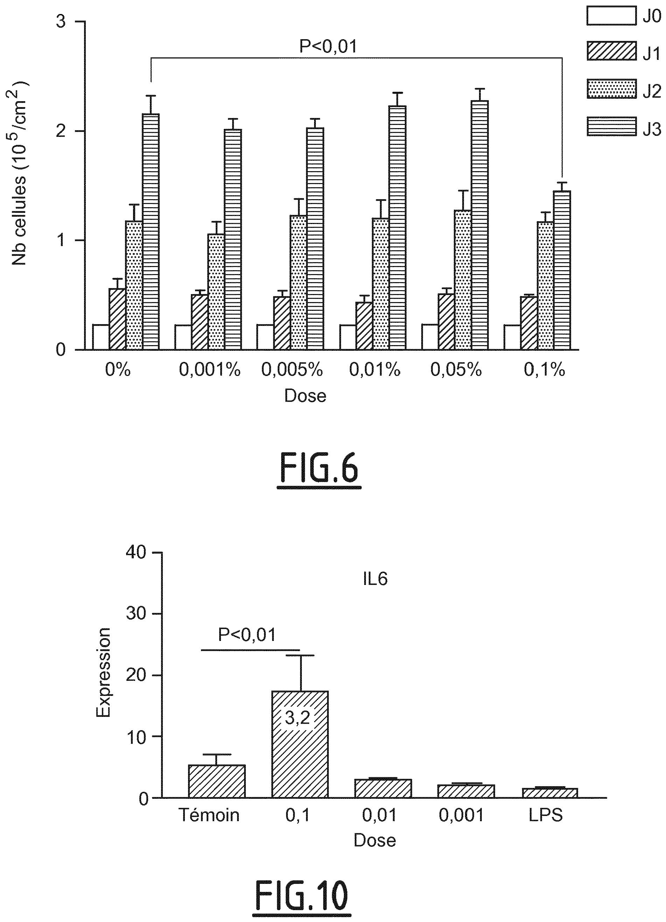

Les résultats des figures 4 à 6 démontrent que la dose maximale non toxique est de 0,1% pour EA1 et EA2 et de 0.5% pour EA3.

La stimulation des cellules IPEC-1 différenciées par les extraits d'algues selon l'invention EA1, EA2 et EA3 est testée 4h à 37°C (environ 10 jours après la confluence) à J15.

Trois doses (DM (dose maximale), DM/10, DM/100) sont testées en comparaison avec du LPS bactérien (contrôle positif) et du milieu de différenciation (contrôle négatif).
Les solutions d'extrait d'algues selon l'invention EA1, EA2 et EA3 obtenues par le procédé indiqué dans l'exemple 1 sont préparées à 0,1 % en DMEM/F12 complet. Pour cela, 0,02g sont repris dans 20 mL et une stérilisation par filtration 0,2 µm est effectuée. Des dilutions successives sont ensuite opérées comme indiqué dans le tableau 4 ci-dessous.

**Tableau 4**

| **Préparation solutions d'extrait d'algues** | | | |
|---|---|---|---|
| | DM | DM/10 | DM/100 |
| | 0,1% | 0,01% | 0,001% |
| dilution désirée | | 10 | 10 |
| dilution obtenue | | 10 | 10 |
| volume dilution précédente | | 2 | 2 |
| volume milieu | | 18 | 18 |
| volume total | 20 | 20 | 20 |
| reste | 18 | 18 | 20 |

La quantité d'extraits d'algues par cellule est indiquée dans le tableau 5 ci-dessous.

**Tableau 5**

| **Dose** | **Volume** | **Qté extrait** | **Nb cellules** | **Qté extrait/cellule** | | |
|---|---|---|---|---|---|---|
| **g/100ml** | **ml/puits** | **mg/puits** | **par puits** | **mg** | **µg** | **ng** |
| 0,001 | 2 | 0,02 | 4,0E+05 | 5E-08 | 0,00005 | 0,05 |
| 0,01 | 2 | 0,2 | 4,0E+05 | 5,0E-07 | 0,0005 | 0,5 |
| 0,1 | 2 | 2 | 4,0E+05 | 5,0E-06 | 0,0050 | 5,0 |

La solution de LPS (SIGMA O111 :B4) est ensuite préparée (dose : 50 ng/10⁵ cellules). 200ng de LPS/puits sous un volume de 2 mL sont déposés. Le tableau 6 ci-dessous indique les différentes concentrations préparées.

**Tableau 6**

| **Préparation solutions LPS** | | | |
|---|---|---|---|
| | 1mg/ml | 1000ng/ml | 100ng/ml |
| dilution désirée | | 1000 | 10 |
| dilution obtenue | | 1000 | 200 |
| vol dilution précédente | | 0,001 | 0,1 |
| volume milieu | 1 | 0,999 | 19,9 |
| volume total | 1 | 1,000 | 20,0 |
| Reste (ml) | 1 | 1 | 20 |

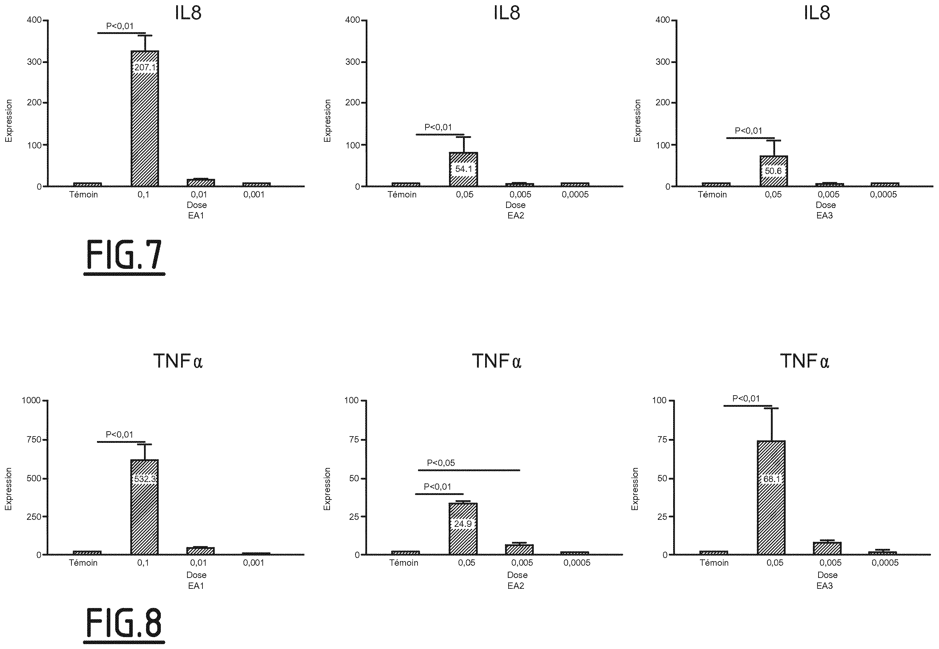

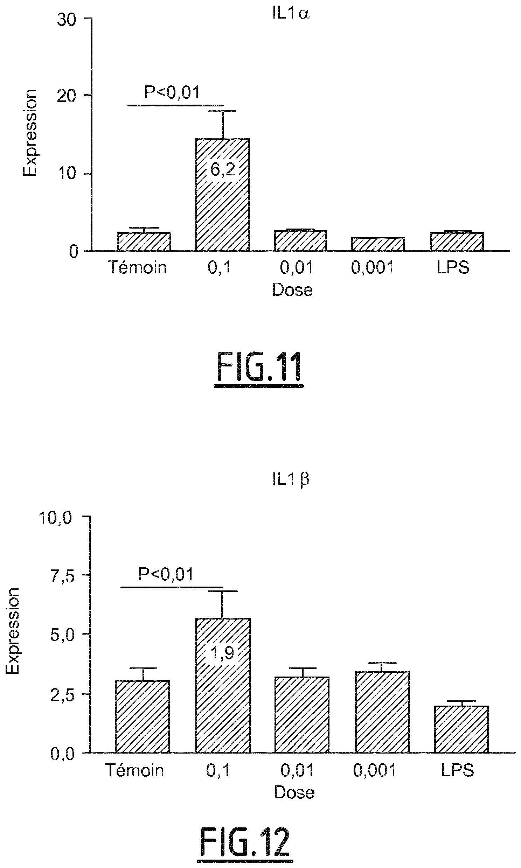

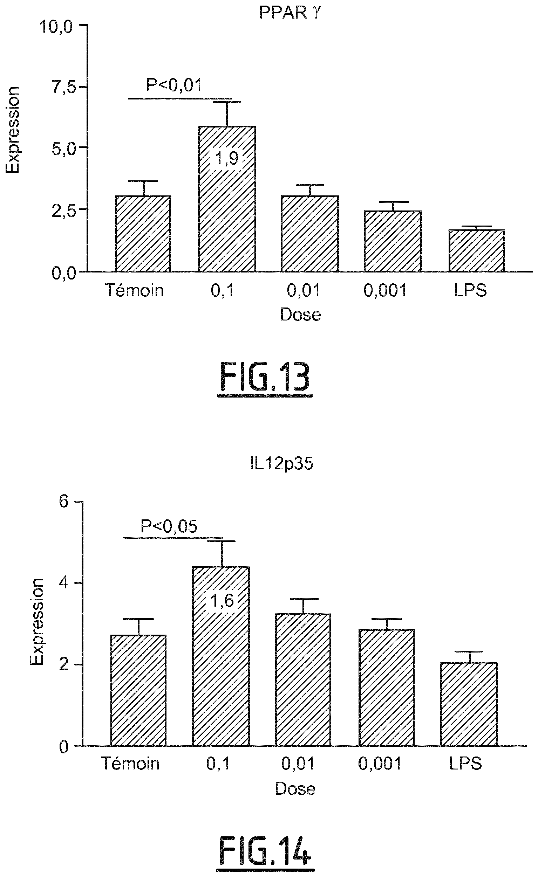

Dans les plaques, on dépose 2 mL de milieu à tester sur chaque filtre et 3 mL en dessous. Le tout est ensuite incubé 4h à 37°C sous 5% de CO₂.
Les cellules IPEC-1 traitées sont ensuite récoltées pour analyse selon le protocole suivant :
1. les puits sont lavés deux à trois fois avec 1 mL d'eau physiologique tamponnée,
2. les cellules sont reprises dans 350 µl de Buffer RA1 (kit Macherey-Nagel), et
3. un transfert dans des tubes Eppendorf, numérotés de 1 à 15 est effectué.
Les ARN sont ensuite extraits en suivant les instructions du fabricant du kit Macherey-Nagel NucleoSpin RNA II, Ref 740955.250, Lot 1211/004.
Les ARNs obtenus sont ensuite transcrits en ADN par reverse transcription selon le protocole suivant :
1. pour chaque échantillon, calculer le volume à prendre pour traiter 1 µg,
2. déposer ce volume dans chaque puits d'une barrette posée sur bloc réfrigéré,
3. ajouter le volume d'eau nécessaire QSP 9 µLI,
4. ajouter 1,3 µL d'oligo dT_ancré 100 µM Sample 3154325 Eurogentec,
5. incuber 10 minutes à 65°C sur GeneAmp PCR System 9700 (USER Khalid),
6. pendant l'incubation préparer le mix dans un tube conique 1,5 mL (pour un échantillon) sur la glace :
   i. 4 µL de tampon 5X
   ii. 2 µL de dNTP 20 mM (Eurogentec)
   iii. 1,9 µL d'eau milliQ
   iv. au dernier moment ajouter 0,8 µL d'enzymes MuMLV 25 U/µl (ME-0125-400) (Eurogentec)
7. sur chaque échantillon ajouter 8,7 µL de mix pour un volume final de 20 µL,
8. poser la barrette sur bloc réfrigéré,
9. incuber 90 minutes à 37°C pour la polymérisation et 5 minutes à 93°C pour inactiver l'enzyme à la fin de la réaction sur GeneAmp PCR System 9700, et
10. congeler à -20°C dans une boite ADN(1)MO.
Les échantillons sont ensuite analysés en qPCR (tests one way ANOVA, différences testées par non paramétriques Dunnet).
L'effet de l'extrait EA1 sur l'expression d'IL8, de TNF-α, de CCL20, d'IL6, d'IL1 a, d'IL1 β, de PPARγ et d'IL 12p35 est testé.
L'effet des extraits EA2 et EA3 sur l'expression d'IL8, de TNF-α et de CCL20 est testé. Les résultats sont indiqués dans les figures 7 à 14.
Ils montrent une croissance de l'expression de l'ARNm des différentes cytokines testées.

L'activité immunostimulatrice d'un extrait d'algues selon l'invention est également évaluée en étudiant la stimulation des facteurs de l'immunité par ELISA sur des cellules IPEC-1 différenciées.
Les deux kits commerciaux utilisés sont le Kit Duo set porcine CXCL8/IL8 (réf : DY535) et le Kit Duo set porcine TNF-α (réf : DY690B) fournis par la société R&D Systems. Les deux kits suivent le même protocole en utilisant un anticorps de capture, un anticorps et un système de détection et des protéines standards spécifiques à chaque interleukine.

### Culture cellulaire et incubation avec les extraits EA1 et EA3 pour la production des cytokines

Les cellules IPEC-1 (0,25×10⁵ cellules/cm²) sont cultivées sur des inserts de culture pendant 3 jours jusqu'à l'obtention d'une confluence cellulaire. Ensuite, le sérum de veau fœtal est remplacé par de la dexaméthasone à 10⁻⁷M pour différencier les cellules pendant une période de 10-14 jours. Trois puits de culture sont incubés pendant 24h à 37°C en présence des extraits EA1 et EA3. Les témoins sont constitués de cultures cellulaires sans extraits d'algues. Les surnageants de culture au-dessus et en-dessous des inserts sont collectés et stockés à - 80°C en tube Eppendorf jusqu'à leur dosage.

### Dosage d'IL8 et de TNF- α par ELISA

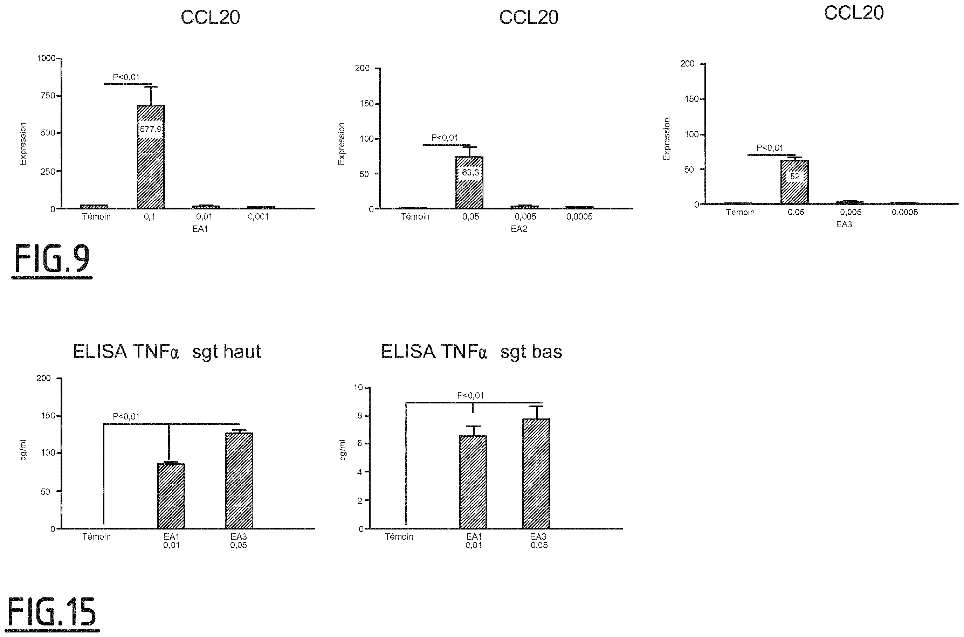

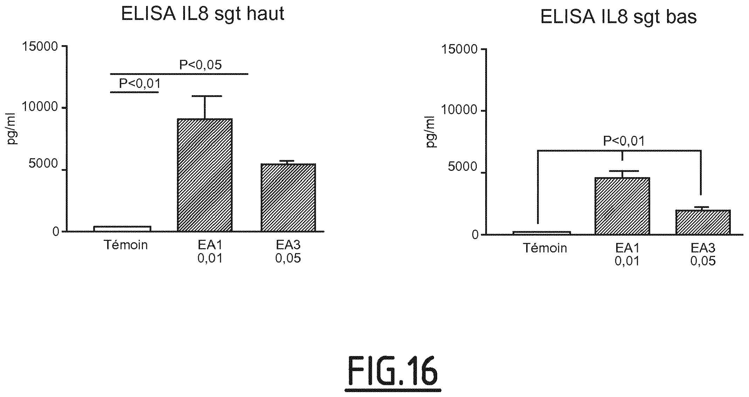

Les anticorps de capture (anti IL-8 ou anti TNF-α de souris), dilués 1/180 dans du PBS, sont immobilisés à raison de 100 µL/puits pendant la nuit à température ambiante. Trois lavages sont effectués pour éliminer les anticorps non fixés avec 400 µL/puits de PBS contenant 0.05% de Tween (tampon de lavage). Ensuite, une étape de saturation est réalisée pour bloquer les sites de fixation non-spécifiques et éviter ainsi que les protéines à tester ne se fixent au plastique. Pour cela, 300 µL/puits de tampon PBS contenant 1% de Sérum Albumine Bovine (BSA) sont ajoutés. Les plaques sont incubées pendant 1 h à température ambiante puis lavées trois fois avec 400 µL/puits de tampon de lavage. Les protéines standards (IL8 et TNF-α recombinantes) sont diluées au demi dans un tampon PBS contenant 1% de BSA pour avoir des concentrations de l'ordre de 4000, 2000, 1000, 500, 250, 125 pg/ml. Un volume de 100 µL d'échantillon de surnageant pur et de protéines standards sont déposés par puits puis incubés pendant 2h à température ambiante. Après une étape de trois lavages avec 400 µL/puits de tampon, les plaques sont incubées en présence de 100 µL/puits d'anticorps de détection couplé à la biotine dilué au 1/180 avec du tampon de dilution contenant 2% sérum de chèvre décomplémenté. Après une incubation de 2h à température ambiante, les plaques sont lavées trois fois avec du tampon de lavage puis incubées avec 100 µL de conjugué *Streptavidine-HRP* pendant 20 minutes à température ambiante à l'abri de la lumière. Après trois lavages avec du tampon de lavage, les puits sont incubés avec 100 µL de substrat (v/v réactif A et B) pendant 20 minutes à température ambiante dans l'obscurité. La réaction HRP-substrat est arrêtée avec 50 µL/puits d'une solution stop et la lecture de la DO (densité optique) est effectuée à 450 nm avec un lecteur ELISA Labsystems Multiskan RC.
Les résultats sont indiqués dans les figures 15 et 16.
Ils montrent une croissance de l'expression protéique des différentes cytokines testées.

### CONCLUSION

L'administration d'un extrait d'algues selon la présente invention met ainsi en évidence un effet immunostimulateur du produit.

## Revendications

1. Extrait d'algues de l'ordre des ulvales, en particulier extrait d'algues vertes de type *Ulva,* comprenant des polysaccharides polyanioniques sulfatés et non sulfatés dont la taille est inférieure ou égale à 50 kDa, à l'exclusion de polysaccharides polyanioniques sulfatés et non sulfatés dont la taille discriminée par ultrafiltration est supérieure à 50 kDa, pour son utilisation pour moduler la réponse immunitaire chez un être humain ou un animal.

2. Extrait d'algues pour son utilisation selon la revendication 1, dans laquelle lesdits polysaccharides comprennent du mannose et/ou de l'arabinose, en particulier du mannose.

3. Extrait d'algues pour son utilisation selon la revendication 2, dans laquelle lesdits polysaccharides comprennent au moins 0.005% de mannose et/ou au moins 0.005% d'arabinose, en poids par rapport au poids de la matière sèche totale de l'extrait d'algues.

4. Extrait d'algues pour son utilisation selon la revendication 3, dans laquelle lesdits polysaccharides comprennent du mannose en une quantité allant de 0.01 à 0.50% et/ou de l'arabinose en une quantité allant de 0.01 à 0.5%, en poids par rapport au poids de la matière sèche totale de l'extrait d'algues.

5. Extrait d'algues pour son utilisation selon l'une quelconque des revendications 1 à 4, dans laquelle lesdits polysaccharides comprennent en outre:
- du galactose;
- du glucose;
- du rhamnose;
- du xylose; et
- de l'acide glucuronique.

6. Extrait d'algues pour son utilisation selon la revendication 5, dans laquelle lesdits polysaccharides comprennent :
- de 0.05 à 0.5% de galactose en poids par rapport au poids de la matière sèche totale de l'extrait d'algues;
- de 0.005 à 0.5% de glucose en poids par rapport au poids de la matière sèche totale de l'extrait d'algues;
- de 2 à 15 % de rhamnose en poids par rapport au poids de la matière sèche totale de l'extrait d'algues;
- de 0.1 à 1% de xylose en poids par rapport au poids de la matière sèche totale de l'extrait d'algues; et
- de 1 à 7% d'acide glucuronique en poids par rapport au poids de la matière sèche totale de l'extrait d'algues.

7. Extrait d'algues de l'ordre des ulvales, en particulier extrait d'algues vertes de type *Ulva,* susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins; et
f) le jus de filtration obtenu à l'étape e) est concentré puis séché ;
pour son utilisation pour moduler la réponse immunitaire chez un être humain ou un animal.

8. Extrait d'algues pour son utilisation selon l'une quelconque des revendications 1 à 7, pour stimuler la réponse immunitaire chez un être humain ou animal, en particulier au niveau du système immunitaire intestinal.

9. Extrait d'algues pour son utilisation selon l'une quelconque des revendications 1 à 8, dans laquelle l'extrait d'algues induit l'expression de molécules d'adhésion et de chimiokines, notamment les IL8 et/ou IL-1α et/ou IL1-β et/ou IL6 et/ou TNF- α et/ou CCL20.

10. Extrait d'algues pour son utilisation selon l'une quelconque des revendications 1 à 9, en tant que complément à la vaccination.

11. Extrait d'algues pour son utilisation selon l'une quelconque des revendications 1 à 10 pour le traitement et/ou la prévention des pathologies infectieuses, notamment les pathologies infectieuses porcines choisies parmi : parvovirose, rouget, rhinite infectieuse, grippe (influenza), circovirose porcine, mycoplasmose et colibacillose ; les pathologies infectieuses aviaires choisies parmi : maladie de Marek, maladie de Newcastle, bronchite infectieuse, maladie de Gumboro, variole aviaire, mycoplasmose, anémie infectieuse aviaire, laryngotrachéite infectieuse, EDS et grippe aviaire ; les pathologies infectieuses bovines choisies parmi: BVD (diarrhées virales bovine), bronchopneumonie enzootique, IBR, herpes virose, clostridiose, colibacillose, fièvre catarrhale, coronavirose, rotavirose et rhinotrachéite ; et les pathologies infectieuses aquacoles choisies parmi: nécrose hématopoïétique, vibriose, furonculose, nécrose pancréatique infectieuse et anémie infectieuse.

12. Extrait d'algues pour son utilisation selon l'une quelconque des revendications 1 à 11, dans laquelle l'extrait d'algues est compris dans une composition pharmaceutique.

13. Extrait d'algues pour son utilisation selon l'une quelconque des revendications 1 à 10, dans laquelle l'extrait d'algues est compris dans une composition alimentaire.

14. Extrait d'algues pour son utilisation selon la revendication 12, dans laquelle l'extrait d'algues est administré à une dose chez l'humain comprise entre 0,1 et 100 mg/kg ou à une dose chez l'animal comprise entre 1 et 200 mg/kg.

15. Extrait d'algues pour son utilisation selon l'une quelconque des revendications 1 à 14, dans laquelle les polysaccharides polyanioniques sulfatés et non sulfatés ont une taille discriminée par ultrafiltration inférieure ou égale à 15 kDa.

16. Extrait d'algues pour son utilisation selon l'une quelconque des revendications 1 à 6, dans laquelle ledit extrait d'algues est susceptible d'être obtenu par un procédé de préparation dans lequel:
a) les algues sont lavées et dessablées ;
b) lesdites algues sont broyées ;
c) la phase solide du broyat est séparée de sa phase liquide ;
d) ladite phase liquide est clarifiée ;
e) le jus obtenu à l'étape d) est ultrafiltré sur une membrane de 50 kDa ou moins; et
f) le jus de filtration obtenu à l'étape e) est concentré puis séché.

17. Extrait d'algues pour son utilisation selon l'une des revendications 7 ou 16, dans laquelle le jus obtenu à l'étape d) du procédé de préparation est ultrafiltré sur une membrane de 15 kDa ou moins.

18. Extrait d'algues pour son utilisation selon l'une quelconque des revendications 7, 16 ou 17, dans laquelle l'extrait d'algues obtenu à l'étape f) du procédé de préparation est purifié, notamment par ultrafiltration.

## Patentansprüche

1. Algenextrakt der Ulvalen-Art, insbesondere Extrakt von Grünalgen des *Ulva-*Typs, aufweisend sulfatierte und nicht sulfatierte polyanionische Polysaccharide, deren Größe kleiner oder gleich 50 kDa ist, unter Ausschluß von sulfatierten und nicht sulfatierten polyanionischen Polysacchariden, deren Größe, die mittels Ultrafiltration diskriminiert wird, über 50 kDa ist, zur Verwendung zum Modulieren der Immunantwort bei einem Menschen oder einem Tier.

2. Algenextrakt zur Verwendung nach Anspruch 1, wobei die Polysaccharide Mannose und / oder Arabinose, insbesondere Mannose, aufweisen.

3. Algenextrakt zur Verwendung nach Anspruch 2, wobei die Polysaccharide mindestens 0,005 Gew.-% Mannose und / oder mindestens 0,005 Gew .-% Arabinose aufweisen, bezogen auf das Gewicht der Gesamttrockenmasse des Algenextrakts.

4. Algenextrakt zur Verwendung nach Anspruch 3, wobei die Polysaccharide Mannose in einer Menge im Bereich von 0,01 bis 0,50 Gew.-% und / oder Arabinose in einer Menge im Bereich von 0,01 bis 0,5 Gew.-% aufweisen, bezogen auf das Gewicht der Gesamttrockenmasse des Algenextrakts.

5. Algenextrakt zur Verwendung nach irgendeinem der Ansprüche 1 bis 4, wobei die Polysaccharide ferner aufweisen:
- Galaktose;
- Glukose;
- Rhamnose;
- Xylose; und
- Glucuronsäure.

6. Algenextrakt zur Verwendung nach Anspruch 5, wobei die Polysaccharide aufweisen:
- 0,05 bis 0,5 Gew.-% Galaktose bezogen auf das Gewicht der Gesamttrockenmasse des Algenextrakts;
- 0,005 bis 0,5 Gew.-% Glukose bezogen auf das Gewicht der Gesamttrockenmasse des Algenextrakts;
- 2 bis 15 Gew.-% Rhamnose bezogen auf das Gewicht der Gesamttrockenmasse des Algenextrakts;
- 0,1 bis 1 Gew.-% Xylose bezogen auf das Gewicht der Gesamttrockenmasse des Algenextrakts; und
- 1 bis 7 Gew.-% Glucuronsäure bezogen auf das Gewicht der Gesamttrockenmasse des Algenextrakts.

7. Algenextrakt der Ulvalen-Art, insbesondere Extrakt von Grünalgen des *Ulva-*Typs, der durch ein Herstellungsverfahren erhalten werden kann, bei welchem:
a) die Algen gewaschen und entsandet werden;
b) die Algen gemahlen werden;
c) die feste Phase des Mahlgutes dessen flüssigen Phase getrennt wird;
d) die flüssige Phase geklärt wird;
e) der in Schritt d) erhaltene Saft mittels einer Membran von 50 kDa oder weniger ultrafiltriert wird; und
f) der in Schritt e) erhaltene Filtrationssaft konzentriert dann getrocknet wird; zur Verwendung zum Modulieren der Immunantwort bei einem Menschen oder einem Tier.

8. Algenextrakt zur Verwendung nach irgendeinem der Ansprüche 1 bis 7 zur Stimulierung der Immunantwort bei einem Menschen oder Tier, insbesondere im Darm-Immunsystem.

9. Algenextrakt zur Verwendung nach irgendeinem der Ansprüche 1 bis 8, wobei der Algenextrakt die Expression von Adhäsionsmolekülen und Chemokinen induziert, insbesondere IL8 und / oder IL-1α und / oder IL1-β und / oder IL6 und / oder TNF-α und / oder CCL20.

10. Algenextrakt zur Verwendung nach irgendeinem der Ansprüche 1 bis 9 als Ergänzung zur Impfung.

11. Algenextrakt zur Verwendung nach irgendeinem der Ansprüche 1 bis 10 zur Behandlung und / oder Vorbeugung von Infektionskrankheiten, insbesondere von Schweineinfektionskrankheiten ausgewählt aus: Parvovirose, Rotbarbe, infektiöser Rhinitis, Grippe (Influenza), Schweinezirkovirus, Mykoplasmose und Colibacillose; Vogel-Infektionskrankheiten ausgewählt aus: Marek-Krankheit, Newcastle-Krankheit, infektiöser Bronchitis, Gumboro-Krankheit, Vogelpocken, Mykoplasmose, infektiöser Vogelanämie, infektiöser Laryngotracheitis, EDS und Geflügelpest; bovinen Infektionskrankheiten ausgewählt aus: BVD (boviner viraler Diarrhöe), enzootischer Bronchopneumonie, IBR, Herpesvirose, Clostridiose, Colibacillose, Blauzungenkrankheit, Coronavirose, Rotavirose und Rhinotracheitis; und Aquakultur-Infektionskrankeheiten ausgewählt aus: hämatopoetischer Nekrose, Vibriose, Furunkulose, infektiöser Pankreasnekrose und infektiöser Anämie.

12. Algenextrakt zur Verwendung nach irgendeinem der Ansprüche 1 bis 11, wobei der Algenextrakt in einer pharmazeutischen Zusammensetzung enthalten ist.

13. Algenextrakt zur Verwendung nach irgendeinem der Ansprüche 1 bis 10, wobei der Algenextrakt in einer Lebensmittelzusammensetzung enthalten ist.

14. Algenextrakt zur Verwendung nach Anspruch 12, wobei der Algenextrakt bei Menschen in einer Dosis zwischen 0,1 und 100 mg / kg oder bei Tieren in einer Dosis zwischen 1 und 200 mg / kg verabreicht wird.

15. Algenextrakt zur Verwendung nach irgendeinem der Ansprüche 1 bis 14, wobei die sulfatierten und nicht-sulfatierten polyanionischen Polysaccharide eine Größe, die mittels Ultrafiltration diskriminiert wird, von weniger als oder gleich 15 kDa haben.

16. Algenextrakt zur Verwendung nach irgendeinem der Ansprüche 1 bis 6, wobei der Algenextrakt durch ein Herstellungsverfahren erhalten werden kann, bei welchem:
a) die Algen gewaschen und entsandet werden;
b) die Algen gemahlen werden;
c) die feste Phase des Mahlgutes dessen flüssigen Phase getrennt wird;
d) die flüssige Phase geklärt wird;
e) der in Schritt d) erhaltene Saft mittels einer Membran von 50 kDa oder weniger ultrafiltriert wird; und
f) der in Schritt e) erhaltene Filtrationssaft konzentriert dann getrocknet wird.

17. Algenextrakt zur Verwendung nach einem der Ansprüche 7 oder 16, wobei der in Schritt d) des Herstellungsverfahrens erhaltene Saft auf einer Membran von 15 kDa oder weniger ultrafiltriert wird.

18. Algenextrakt zur Verwendung nach einem der Ansprüche 7, oder 17, wobei der in Schritt f) des Herstellungsverfahrens erhaltene Algenextrakt gereinigt wird, insbesondere durch Ultrafiltration.

## Claims

1. An extract of algae from the order of ulvales, in particular an extract of green algae of the *Ulva* type, comprising sulfated and non-sulfated polyanionic polysaccharides for which the size is less than or equal to 50 kDa, excluding sulfated and non-sulfated polyanionic polysaccharides for which the size discriminated by ultrafiltration is greater than 50 kDa, for its use for modulating the immune response in a human being or an animal.

2. The algae extract for its use according to claim 1, wherein said polysaccharides comprise mannose and/or arabinose, in particular mannose.

3. The algae extract for its use according to claim 2, wherein said polysaccharides comprise at least 0.005% of mannose and/or at least 0.005% of arabinose, by weight based on the weight of the total dry material of the algae extract.

4. The algae extract for its use according to claim 3, wherein said polysaccharides comprise mannose in an amount ranging from 0.01 to 0.50% and/or arabinose in an amount ranging from 0.01 to 0.5%, by weight based on the weight of the total dry material of the algae extract.

5. The algae extract for its use according to any of claims 1 to 4, wherein said polysaccharides further comprise:
- galactose;
- glucose;
- rhamnose;
- xylose; and
- glucuronic acid.

6. The algae extract for its use according to claim 5, wherein said polysaccharides comprise:
- from 0.05 to 0.5% of galactose by weight based on the weight of the total dry material of the algae extract;
- from 0.005 to 0.5% of glucose by weight based on the weight of the total dry material of the algae extract;
- from 2 to 15 % of rhamnose by weight based on the weight of the total dry material of the algae extract;
- from 0.1 to 1% of xylose by weight based on the weight of the total dry material of the algae extract; and
- from 1 to 7% of glucuronic acid by weight based on the weight of the total dry material of the algae extract.

7. An extract of algae from the order of ulvales, in particular an extract of green algae of the *Ulva* type, which may be obtained by a preparation method wherein:
a) the algae are washed and cleared of sand;
b) said algae are milled;
c) the solid phase of the milled material is separated from its liquid phase;
d) said liquid phase is clarified;
e) the juice obtained in step d) is ultrafiltered on a membrane of 50 kDa or less; and
f) the filtration juice obtained in step e) is concentrated and then dried;
for its use for modulating the immune response in a human being or an animal.

8. The algae extract for its use according to any of claims 1 to 7, for stimulating the immune response in a human being or animal, in particular at the intestinal immune system.

9. The algae extract for its use according to any of claims 1 to 8, wherein the algae extract induces the expression of adhesion molecules and of chemokines, notably IL-8 and/or IL-1α and/or IL1-β and/or IL-6 and/or TNF-α and/or CCL20.

10. The algae extract for its use according to any of claims 1 to 9, as a supplement to vaccination.

11. The algae extract for its use according to any of claims 1 to 10 for treating and/or preventing infectious pathologies, notably porcine infectious pathologies selected from: parvovirus disease, Erysipela Rhusiopathiae, infectious rhinitis, influenza, porcine circovirus disease, mycoplasma and colibacillosis; avian infectious pathologies selected from: Marek's disease, Newcastle's disease, infectious bronchitis, Gumboro's disease, fowlpox, mycoplasma, avian infectious anemia, infectious laryngotracheitis, EDS and avian influenza; bovine infectious pathologies selected from: BVD (bovine viral diarrheas), enzootic bronchopneumonia, IBR, herpes virus disease, clostridial disease, colibacillosis, bluetongue disease, coronavirus disease, rotavirus disease and rhinotracheitis; and aquaculture infectious pathologies selected from: hematopoietic necrosis, vibriosis, furunculosis, infectious pancreatic necrosis and infectious anemia.

12. The algae extract for its use according to any of claims 1 to 11, wherein the algae extract is comprised in a pharmaceutical composition.

13. The algae extract for its use according to any of claims 1 to 10, wherein the algae extract is comprised in a food composition.

14. The algae extract for its use according to claim 12, wherein the algae extract is administered at a dose in humans comprised between 0.1 and 100 mg/kg or at a dose in animals comprised between 1 and 200 mg/kg.

15. The algae extract for its use according to any of claims 1 to 14, wherein the sulfated and non-sulfated polyanionic polysaccharides have a size of less than or equal to 15 kDa.

16. The algae extract for its use according to any of claims 1 to 6, wherein said algae extract may be obtained by a preparation method wherein:
a) the algae are washed and cleared of sand;
b) said algae are milled;
c) the solid phase of the milled material is separated from its liquid phase;
d) said liquid phase is clarified;
e) the juice obtained in step d) is ultrafiltered on a membrane of 50 kDa or less; and
f) the filtration juice obtained in step e) is concentrated and then dried.

17. The algae extract for its use according to one of claims 7 or 16, wherein the juice obtained in step d) of the preparation method is ultrafiltered on a membrane of 15 kDa or less.

18. The algae extract for its use according to any of claims 7, 16 or 17, wherein the algae extract obtained in step f) of the preparation method is purified, notably by ultrafiltration.
